(19) Europäisches Patentamt
European Patent Office
Office européen des brevets

(11) EP 0 797 652 B1

(12) EUROPEAN PATENT SPECIFICATION

(45) Date of publication and mention of the grant of the patent:
**23.08.2006 Bulletin 2006/34**

(21) Application number: **95941710.6**

(22) Date of filing: **13.12.1995**

(51) Int Cl.:
***C10M 173/02*** *(2006.01)* *C10M 173/02* *(2006.01)*
*C10M 129/40* *(2006.01)* *C10M 133/08* *(2006.01)*
*C10N 30/04* *(2006.01)* *C10N 40/00* *(2006.01)*

(86) International application number:
**PCT/EP1995/004935**

(87) International publication number:
**WO 1996/018709 (20.06.1996 Gazette 1996/28)**

# (54) SOAP-BASED LUBRICANT COMPOSITION FREE FROM COMPLEXING AGENTS

KOMPLEXBILDNERFREIE SCHMIERMITTELZUSAMMENSETZUNG AUF SEIFENBASIS

COMPOSITION LUBRIFIANTE A BASE DE SAVON NE CONTENANT PAS D'AGENTS DE COMPLEXION

(84) Designated Contracting States:
**AT BE CH DE DK ES FR GB GR IE IT LI LU MC NL PT SE**

(30) Priority: **14.12.1994 DE 4444598**

(43) Date of publication of application:
**01.10.1997 Bulletin 1997/40**

(73) Proprietor: **Calvatis GmbH**
**68526 Ladenburg (DE)**

(72) Inventors:
- **SCHOBER, Klaus**
  **D-67259 Heuchelheim (DE)**
- **HERRMANN, Frank**
  **D-68723 Oftersheim (DE)**

(74) Representative: **Maiwald Patentanwalts GmbH**
**Elisenhof,**
**Elisenstrasse 3**
**80335 München (DE)**

(56) References cited:

| | |
|---|---|
| **EP-A- 0 233 774** | **EP-A- 0 591 771** |
| **WO-A-93/18120** | **WO-A-94/04648** |
| **DE-A- 2 302 367** | **US-A- 3 594 411** |
| **US-A- 3 933 660** | **US-A- 3 945 930** |
| **US-A- 5 352 376** | |

- **DATABASE WPI Section Ch, Week 9329 Derwent Publications Ltd., London, GB; Class D21, AN 93-232642 & JP,A,05 156 282 ( KAO CORP) , 22 June 1993**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 797 652 B1

## Description

The invention relates to lubricant compositions which contain soap, yet are free from complexing agents and suitable, in particular, for lubricating conveyor belts in the food and beverage industry.

In the food and beverage industry, e.g. in breweries or bottling operations at mineral water sources, bottle cleaning, filling and labelling is carried out mainly by automation. As a rule, high-performance lubricants need to be used on the bottle conveyor facilities to guarantee problem-free conveying of the bottles. The filling performance of filling equipment has increased over the years as a result of technological progress. Requirements regarding the performance of the belt lubricants have increased correspondingly. In parallel, greater environmental awareness of the users has resulted in a demand for more environmentally and userfriendly chemicals particularly when these are sprayed openly in premises, as is the case with belt lubricants, and where the lubricant liquid dripping off the belt may pass directly into the effluent (waste water) from the plant.

State-of-the-art lubricants for conveyor belts in the food and beverage industry are mainly prepared by diluting, with water, concentrates containing soap, surfactants, fatty acids, alkanol amines and complexing agents.

Such soap-based lubricants are e.g. disclosed in US patents Nos. 3 860 521 and 4 274 973. In US patent 4 604 220, a soap-based conveyor cleaner and conveyor belt lubricant concentrate is disclosed, which bases on $C_{12}$ to $C_{18}$ alpha olefin sulfonates and corresponding polyether derivatives. This alpha olefin sulfonate can be combined with anionic or nonionic surfactants, or can be combined with fatty acid soap lubricant compositions to enhance the acid resistance of such soaps.

Combinations of fatty acid soap and alpha olefin sulfonates are, however, not suitable for applications where hard water must be used. This combination appears not to have had any commercial success. Combinations of soaps and alpha olefin sulfonates or alpha olefin polyether sulfonates are not claimed as forming part of the instant invention.

More recently, it has been suggested in WO 92/13049, that fatty acid salts of specific fatty acid diamines can be used in combination with anionic and/or nonionic surfactants, complexing agents or alkyl sulfonates as belt conveyor lubricants, specifically for PET and PC containers, but not for glass. These compounds can contain fatty acid soaps as a solidifying agent, where solid combinations are desired. In these compositions, the diamine and fatty acid contents must be carefully balanced, to provide overall neutralization. All compositions exemplified in WO 92/13049 comprise substantial amounts of EDTA, as a sequestrant or complexing agent. Based on this art, it would appear that generally, and specifically for hard water applications, use of a sequestrant in soap-containing lubricants is unavoidable.

A major disadvantage of soap-containing lubricants is their sensitivity to hard water. Soaps tend to react with water hardness forming salts (mainly $Ca^{2+}$ or $Mg^{2+}$ salts) to form the so-called lime soaps which are difficult to dissolve or even insoluble and strongly reduce the lubrication performance of the lubricant. Frequently, this leads to the formation of deposits on the conveyor belts and finally to operating problems. To avoid this precipitation of lime soap, strong complexing agents (such as EDTA) are added to soap-containing lubricants or the application needs to be restricted to very soft water (which, however, is not generally available). For ecological reasons, the use of strong complexing agents in lubricants which pass into the waste water discharged from the plant is undesirable, since these complexing agents have poor biological degradability. EDTA especially is practically not biodegradable.

There have been attempts in the art at solving this problem, either by use of biodegradable, yet sufficiently strong complexing agents for soap-based lubricants, or through alternative lubricants which comprise no soap. Generally, these alternative lubricants attempt to replace soap with specific amino compounds.

In EP-B1 0 044 458, a soap-free lubricant composition is disclosed which contains no complexing agent. The lubricant composition comprises alkyl polyether carboxylic acid salts combined with acyl sarcosinates.

From EP-B1 0 260 508, a method for lubricating a conveyor belt is known, which comprises the steps of lubricating the belt with a soap-free lubricant based on neutralized primary fatty amines, and cleaning the belt with cationic cleaning agents or organic acids. The reason for this is that the amines used for lubrication form precipitates with anions, which strongly reduce the lubricating effect and can clogg spray nozzles etc.

From EP-B1 0 372 628, and EP-A1 0 538 916 another substitute for soap-based lubricants is known, which is based on di- or polyalkyl amines or corresponding di- or polyalkyl aminoalkyl carboxylic acids. The aqueous lubricating solutions made from these compounds are used at pH-values between 5 and 8. The compositions contain no soap and thus require no complexing agent.

EP-B1 0 384 282 (which has been consolidated with EP 0 593 420) discloses the use of secondary and/or tertiary amines and/or salts thereof in lubricating formulations for PET or PC bottles. Generally, the amines are used as such, without the addition of surfactant, complexing agent or cosolvent.

WO 94/03562 discloses the use of polyamine derivatives of fatty amines and/or salts thereof. It is claimed that these amines are per se not very sensitive to process water anions such as sulfate, bicarbonate etc. The examples show that the lubricants of WO 94/03562 were only tested at low alkalinity (pH below 8), and were not tested with respect to their water hardness cation resistance. As such, the fatty amine salt-based lubricants of this art would not be expected to provide sufficient lubrication combined with the absence of precipitation problems in hard water application.

In DE-C2 42 44 536, it is suggested to base a soap-free lubricant on mainly two components, one of which is an alkyl diamine, optionally neutralized with an organic acid, and the other of which is a polyether carboxylic acid. The lubricants can be used between pH 6.5 and 7. The lubricant solution is made from a corresponding concentrate by adding water, from which the hardness forming ions have, before, been removed. It does not appear likely that such a composition could be used at typical application conditions, with pH > 7, to achieve the necessary lubricating effect and, at the same time, avoid precipitation of solids from the lubricant solution.

DE-A1 43 11 237 proposes a specific sequestrant, based on polyasparagic acid and copolymerisates thereof, to overcome the problem that typical complexing agent such as EDTA are not biodegradable.

In DE-A1 43 15 21 (and corresponding EP 0 623 666) of the present applicant, it has been suggested to prepare soap-free lubricants on the basis of polyamines, which can optionally be combined with organic acids, to adjust the pH of the concentrate to between 4 and 8.

US 5,352,376, issued to Gutzmann, is related to a thermoplastic compatible lubricant concentrate containing alkylpolyglycoside suitable for use in lubricating conveyor belts in the transporting of thermoplastic article of manufacture.

DE 2 302 367 discloses a liquid cleaning and lubricating agent which contains fatty acid soap, synthetic anionic-activ material for washing and a vinylether-maleic anhydride copolymers in an aqueous solution.

WO 93/1820 is related to water-soluble lubricants for chain belt conveyors containing a combination of at least one specific *sec*-amine alkyl carboxylic compound, at least one organic carboxylic acid, and water.

EP 0 233 774 discloses a synergistic aqueous lubricant composition which comprises one or more carboxylated surfactants and one or more non-carboxylated surfactants, wherein lime soap dispersion is improved.

JP 2 055 794 is directed to compositions capable of reducing the dynamic frictional force of a belt conveyor in food plants by incorporating a salt of a fatty acid having a specified number of carbon atoms into a surface active agent selected from cationic and ampholytic surface active agents.

The lubricant performance of soap products is usually better as compared to the so-called "synthetic" lubricants based on surfactants, fatty amine acetates etc.

In addition, when used appropriately under open conditions (e.g. in bottle conveyor facilities) soaps have found a high degree of acceptance by the user because of their operating safety. Several of the prior art amino compounds have a microbicidal effect and have not yet been assessed in view of human toxicology. Cases of incompatibility and undesirable odour as well as problems in plant or communal effluent treatment facilities have arisen.

The invention is based on the object of providing lubricants for conveyor belts and claims which are toxicologically and ecologically safe from the user point of view and which, on the one hand, are free from polymers or complexing agents which are not, or only poorly, biodegradable and yet, on the other hand, form no deposits or precipitates even with very hard water. Another object of the invention is to find lubricant compositions that are suitable both for lubricating conveyor belts for glass containers (e.g. beverage bottles) and for conveying plastic containers (e.g. beverage bottles made of PET or PC). The lubricating performance of such lubricants should correspond at least to that provided by state-of-the-art lubricants. The use of the lubricants should be possible, largely independent of the hardness of the water used for the preparing the aqueous lubricating composition.

The invention is inter alia based on the finding that surprisingly, the consistant approach of the prior art in this field is wrong. In the whole of the art, it has been maintained that there are only two kinds of useful lubricant compositions: One is soap-based, but can only be used with typical hardness - containing process waters when combined with substantial amounts of complexing agents; the other is based on amino derivatives and avoids the use of soap completely, at the cost of anion sensitivity of the composition and general lower lubricating performance.

Surprisingly, it has now been found that this prior art prejudice is not supported by the facts. According to the invention, a soap-based lubricant, with the high lubricating efficiency typical of such soap-based products, can be formulated without complexing agents, when instead, the further components claimed and described hereinafter, are used. Some of these are, per se, components of prior art soap-free lubricant compositions; others have never before been disclosed in the art as components of such lubricant compositions.

In the absence of sufficient scientific research, the present inventors do not want to speculate on the exact mechanism by which these co-components prevent the formation of lime soap precipitates or even turbidity without, at the same time, negatively affecting the lubricating efficiency of the soap-based composition. Seeing that the prior art soap-free lubricants are generally markedly inferior to soaps in lubricating efficiency, one would have expected a notable decrease in lubricating efficiency when adding such substances to a soap-based lubricant. No such effect is, however, observed. The effectiveness of the inventive preparations is even more surprising seeing that the co-component used is generally not a sequestrant or complexing agent for water hardness ions such as $Ca^{2+}$ and $Mg^{2+}$. This could lead one to assume that there is a completely different mechanism involved. It is presently assumed that all co-components of this invention are capable of acting as dispersants for soaps which could be formed, especially at higher pH and high degrees of water hardness, from the soap component of the invention's lubricants. The invention's co-components, which are combined with soap based lubricant com-ponents, are therefore hereinafter referred to as "soap dispersants".

Thus, the invention makes it possible, for the first time, to obtain the benefits of a soap-based lubricant without

incurring the disadvantage of having to use a complexing agent or sequestrant to prevent precipitation of poorly soluble or even insoluble compounds formed from the soap component and water hardness ions, especially calcium ions and magnesium ions.

The invention achieves this by providing a lubricant concentrate, especially for preparing an aqueous lubricant composition for belt or chain conveyors as used in the bottling industry, which concentrate comprises a soap component, especially a fatty acid alkali or amino soap, in an amount sufficient to provide lubricating properties to the aqueous lubricant composition, said concentrate further comprising a functional amount of at least one substantially biodegradable nonionic, anionic or amphoteric soap dispersant, which is capable of preventing the precipitation of poorly soluble or insoluble hardness ion compounds of the soap component, without having, itself, any substantial hardness ion complexing or sequestering properties. The inventive concentrate is thus substantially free from hardness ion complexing or sequestering agents. The soap dispersant excludes alkylpolyglycosides.

In the inventive embodiments, the biodegradable soap dispersant of the lubricant concentrate is at least one of an amphoteric alkyl (monoamine or polyamine) carboxylate, or a betaine, especially an alkyl or an alkylamido betaine or sulfobetaine as defined in any one of claims 1 or 3.

Specifically, alkyl monoamine carboxylates and alkyl sulfobetaines as well as alkylamido sulfobetaines have been shown, as will be described in more detail hereinafter, to be highly effective co-components in the invention's soap-based lubricants.

These lubricant concentrates preferably comprise at least one soap dispersant which comprises at least one compound of the general formula (I)

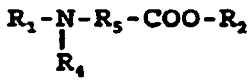 (I)

wherein $R_1$ is a linear, branched, saturated or unsaturated alkyl residue having 8 to 22 carbon atoms or a corresponding alkoxy residue, $R_2$ is Na, K or $N(R_3)_3$, wherein $R_3$ is H, alkyl or hydroxyalkyl, $R_4$ is H, an alkyl, hydroxyalkyl, $R_5$-COO-$R_2$ or

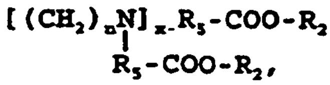

wherein $R_5$ is alkyl or hydroxyalkyl, h is am integer from 1 to 10 and x is an integer from 1 to 50.

More specifically, a presently especially preferred soap dispersant comprises an alkyl monoamine carboxylate corresponding to the general formula (I), wherein $R_1$ is cocoalkyl, $R_4$ is H, $R_5$ is ethylene and $R_2$ is triethanolammonium ion.

Such alkyl monoamine carboxylates can be obtained under the tradename Amphoram CP1 from CECA S.A., France.

Another more specific preferred soap dispersant comprises an alkyl polyamine carboxylate corresponding to the general formula (I), wherein $R_4$ is

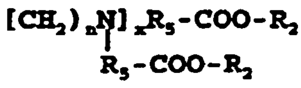

and
and wherein

$R_5$ is methylene or ethylene, preferably methylene
$R_2$ is Na or K, preferably Na
n is 2 to 4, preferably 3 and
x is 1 to 5, preferably 1 to 3.

Such a dispersant can be obtained, under the tradename Amphionic SFB, from Rhone Poulenc, France.

An alternatively especially preferred soap dispersant comprises a betaine of the general formula (II),

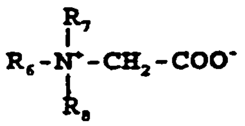 (II)

wherein $R_6$ is cocoalkyl and $R_7$ and $R_8$ are hydroxyethyl.

Such dispersants can be obtained under the tradenames. Tegotain N192 from Goldschmidt, Germany and Amphotensid B4 from Zschimmer und Schwarz, also Germany.

Also alternatively, the soap dispersant can advantageously comprise a sulfobetaine corresponding to the general formula (III)

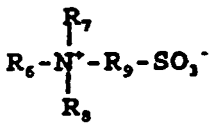 (III)

wherein $R_6$ is lauryl, $R_7$ and $R_8$ are methyl and $R_9$ is 2-hydroxypropyl, or $R_6$ is cocoamidopropyl, $R_7$ and $R_8$ are methyl and $R_9$ is 2-hydroxypropyl.

Such sulfobetaines (or sultaines) can be obtained from Witco/Rewo Chemische Werke GmbH, Germany, under the tradename Rewoteric AM HC, and from Rhone Poulenc, France, under the Mirataine CBS tradename.

To obtain permanently clear application solutions in hard water, it is advantageous for the soap dispersant to be present in excess, in comparison with the soap component. The best results are often achieved by using one or more soap dispersant compounds in a 2 to 4 fold excess over the soap component in the lubricant concentrate. The soap dispersant components are often present in a ratio of approximately 1:1 since then these components develop their maximum activity for the prevention of lime-soap precipitation.

To vary the cleaning effect and the foaming properties of the lubricant compositions according to the invention, the formulations may additionally contain surfactants (usually nonionic or anionic surfactants).

To be able to provide the lubricants according to the invention with germ-reducing properties, the formulation may contain disinfectant or preservatives. To advantageously influence the storage stability and flow properties of the lubricant concentrates, the formulations may additionally contain solubilisers in the form of mono or polyhydric alcohols (usually lower aliphatic alcohols or glycols).

As a rule, the pH of the concentrate is above neutrality. To obtain clear application solutions, it may be advantageous to use alkanol amines such as triethanol amine in a slight excess as the base. The excess base simultaneously has the effect of preventing the pH of the highly dilute lubricant solutions from decreasing too strongly in acidic process water. The pH of the application solutions is usually also in the alkaline range and often above 8. When the soap dispersant comprises a di- or polyamino compound as claimed in EP-B1 0 372 628, the pH is always 8 or higher.

For the preparation of the lubricant concentrates according to the invention, the individual components can generally be used in acid or salt form.

The appropriate amount of water (hard or, where easily available, demineralised water) is introduced into a mixing vessel equipped with a stirrer. Subsequently, the components in the acid or the salt form are added. Generally, this takes the form of addition of a preformed concentrate comprising all components. However, it is of course possible to prepare an aqueous lubricant composition of this invention by adding the individual components to the water one by one. By adding the corresponding base, the dispersion is converted into a clear solution of the concentrate.

Surprisingly, the poor inherent lubricant properties of those inventive soap dispersants which have been used in the art as soap-free lubricants do not negatively affect the good lubricant properties of the soap component while nevertheless preventing the undesirable formation of lime-soap and thus the formation of precipitates and deposits.

Because of the above-mentioned properties, lubricant compositions according to the invention are suitable for the use also outside the food and beverage industry when lubricating problems arise in cases where physiological safety and high biodegradability in association with excellent lubricating properties are desireable or necessary.

The superior properties of the lubricant composition according to the invention, in comparison with the properties of their components, and thus the synergistic effect thus achieved, will now be shown in further detail by way of the following examples, in association with the attached drawing. In the drawing

Fig. 1 represents a diagrammatic representation of a bottle conveyor facility on which the reported experiments were

carried out

Fig. 2 shows a graphic representation of the sliding friction coefficients achieved with different lubricants and lubricant compositions.

For the turbidity tests reported below, a synthetic water with a hardness of 25° German hardness was used which was prepared as follows:

Stock solution 1: 23.63 g $CaCl_2$ x 2 $H_2O$ (0.161 mole) and 3.63 g $MgCl_2$ x 6 $H_2O$ (0.018 mole) were dissolved in 1000 ml of distilled water.

Stock solution 2: 22.50 g $NaHCO_3$ (0.268 mole) and 12.32 g $NaHSO_4$ (0.089 mole) were dissolved in 1000 ml of distilled water.

25° German hardness: 25 ml of stock solution 1 and 25 ml of stock solution 2 were diluted with demineralised water to 1000 ml in a measuring flask.

This synthetic hard water had a pH of approximately 8.3. The water hardness consisted of carbonate hardness (approximately 50%) and of non-carbonate hardness (the remaining 50%).

To determine the lubricating performance of the lubricants according to the invention and their individual components, an in-house bottle conveyor facility was used which is illustrated in the diagrammatic representation in fig. 1. The belt speed and the spray volume per unit of time were kept constant during the measurements. The lubricant solutions were prepared using local (Ladenburg) town water (24° German hardness). 0.2% of the lubricant concentrate were used for the comparative measurements.

Example I

A lubricant composition according to the invention was made up to have the following composition:

Component

A 2% oleic fatty acid triethanol amine salt (soap component according to formula VI)

B 5% N-coconut alkyl-β-alanine triethanol amine salt (first soap dispersant, according to formula I)

C 5% alkyl (C9/11) polyether (5EO) carboxylic acid triethanol amine salt (second soap dispersant, according to formula IV)

D 88% demineralised water.

(B is "Amporam CP1" from CECA S.A., C is "Akypo RO 50" from Chem-Y GmbH).

The above-mentioned lubricant composition did not exhibit any turbidity when diluted in any proportion with the synthetic water at 25° German hardness as described above, over a period of several weeks. Solutions of soap component A only, on the other hand, formed milky turbid precipitates within a short time.

The lubricating effect of the lubricant composition of the Example I (solution 4 in Talbe 1) was determined using the bottle conveying facility (Fig. 1) and compared with the lubricating effect of the individual components and the lubricating effect of an amine acetate, which is a representative belt lubricant according to the state of the art.

Table 1

| Component | Solution 1 | Solution 2 | Solution 3 | Solution 4 | Solution 5 |
|---|---|---|---|---|---|
| A | 2% | | | 2% | |
| B | | 5% | | 5% | |
| C | | | 5% | 5% | |
| Water | 98% | 95% | 95% | 88% | 95% |
| Amine acetate* | | | | | 5% |
| *N-coconutalkyl propylene diamine diacetate. | | | | | |

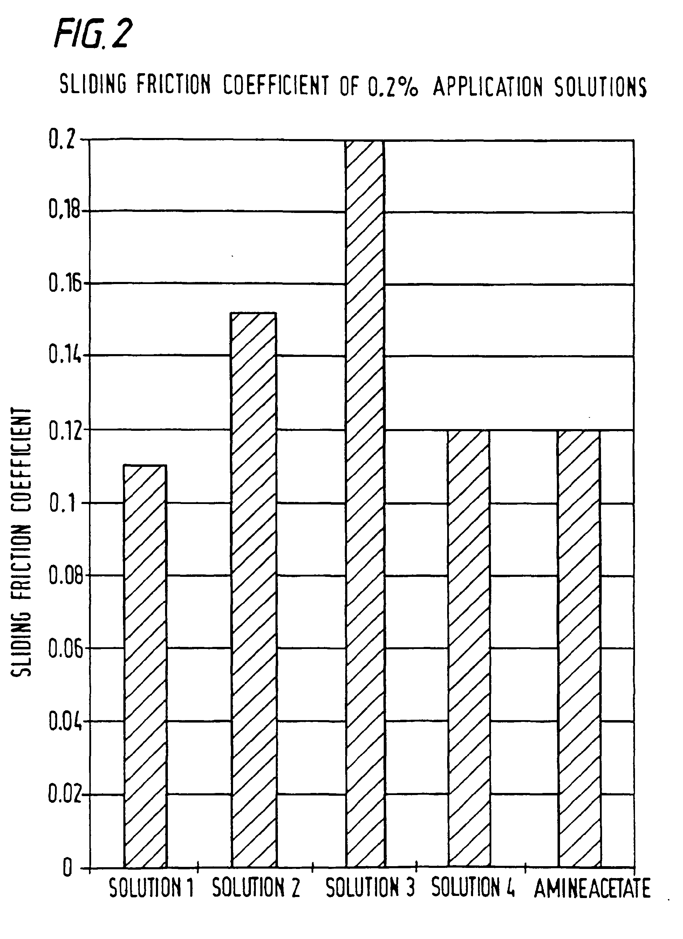

Fig. 2 show the sliding friction coefficient of 0.2% dilutions of solutions 1 to 5. The sliding friction coefficient K represents a measure of the performance of the lubricant and is calculated using the formula

$$K=F/G$$

K sliding friction coefficient
F force measured on the measuring facility of the bottle conveying plant (Fig. 1)
G weight of the bottles used in the test.

The lower the sliding friction coefficient, the greater the lubricating effect. The dilution value of soap solution A was measured with fully demineralised water, whilst the values for solution B, C and D and for the solution of amine acetate were measured using Ladenburg tap water (24° German hardness). Fig. 2 clearly shows that pure soap solution has the lowest sliding friction coefficient (K = 0.11). Whereas solution C of polyether carboxylic acid salt exhibits practically no relevant lubricating effect (K)>0.2), solution B of amphoteric surfactant has a low lubricating effect (K = 0.152) which is insufficient for the purpose of the application according to the invention.

As shown in Fig. 2, the sliding friction coefficient (K = 0.12) of the inventive solution 4 was practically as highg as that of soap solution. This value is comparable to the sliding friction coefficient of a commercial quality amine acetate which represents the present state of the art in this field.

**Examples II - XIV**

Corresponding tests were made, using the following individual soap dispersants of the instant invention, combined singly with a soap component as in Example I:

| Example | Soap dispersant | Tradename | Soap Dispersing Effect |
|---|---|---|---|
| II (Comparative) | fatty alcohol alkoxylate | Synperonic LF/RA 30 | ++ |
| III (Comparative) | alkyl polyglucoside | Glucopon CSUP | + |
| IV (Comparative) | alkyl polyether carbonic acid | Akypo RO 50 | ++ |
| V (Comparative) | alkyl polyether sulfate | Manro BES | ++ |
| VI (Comparative) | alkyl polyether phosphate | Marlopor FC | + |
| VII (Comparative) | alkyl polyether sulfosuccinate | Tensuccin HM935 | + |
| VIII (Comparative) | alkylamido polyether sulfosuccinate | Lankropol KS6 | + |
| IX | alkyl mono-amine carboxylate | Amphoram CP1 | ++ |
| X | alkyl polyamine carboxylate | Amphionic SFB | + |
| XI | alkyl betaine | Tegotain N192 | + |
| XII | alkylamido betaine | Amphotensid B4 | + |
| XIII | alkyl sulfobetaine | Rewoteric AM HC | ++ |
| XIV | alkylamido sulfobetaine | Mirataine CBS | ++ |

All Examples exhibited good to very good soap dispersing effect, shown by very slight or no turbidity after several weeks, even when rather alkaline, very hard local tap water was used. The lubricating properties of the combinations of soap and soap dispersant of Examples II to XIV were generally comparable to those of the soap component as such.

## Claims

1. Use of a lubricant concentrate for preparing an aqueous lubricant composition for belt or chain conveyors used in the food industry, comprising a soap component, especially a fatty acid alkali or amino soap, in an amount sufficient

to provide lubricating properties to the aqueous lubricant composition, and, in case, comprising minor amounts of further customary lubricant concentrate components,
**characterized in that** the concentrate is substantially free from hardness ion complexing or sequestering agents, and that the lubricant concentrate comprises a substantially biodegradable, nonionic, anionic or amphoteric soap dispersant preventing the precipitation of poorly soluble or insoluble hardness ion compounds of the soap component without having any substantial hardness ion complexing or sequestering properties, and that the lubricant concentrate is diluted in hard water, wherein the concentrate comprises, as the biodegradable soap dispersant, at least one of soap dispersant selected from the following compounds:

a) an amphoteric alkyl monoamine,
b) a polyamine carboxylate
c) a betaine of the general formula (II)

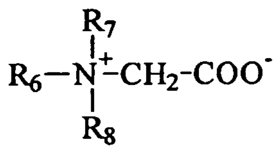

(II)

wherein $R_6$ is cocoalkyl, and $R_7$ and $R_8$ are hydroxyethyl; or
d) a sulfobetaine corresponding to the general formula (III)

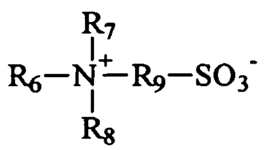

(III)

wherein $R_6$ is lauryl or cocoamidopropyl, $R_7$ and $R_8$ are methyl and $R_9$ is 2-hydroxypropyl;

**2.** Use of an aqueous lubricant composition as defined in claim 1, and having a pH of 8 or higher, to lubricate a conveyor belt or chain.

**3.** Lubricant concentrate, especially for preparing an aqueous lubricant composition for belt or chain conveyors used in the food industry, comprising a soap component, especially a fatty acid alkali or amino soap, in an amount sufficient to provide lubricating properties to the aqueous lubricant composition, and, in case, comprising minor amounts of further customary lubricant concentrate components,
**characterized in that** the concentrate is substantially free from hardness ion complexing or sequestering agents, and comprises a substantially biodegradable, nonionic, anionic or amphoteric soap dispersant preventing the precipitation of poorly soluble or insoluble hardness ion compounds of the soap component without having any substantial hardness ion complexing or sequestering properties, wherein the soap dispersant is selected from the following compounds:

a) at least one compound of the general formula (I)

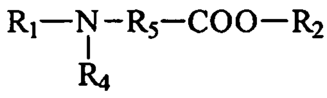

(I)

wherein $R_1$ is a linear, branched, saturated or unsaturated alkyl residue having 8 to 22 carbon atoms or a corresponding alkoxy residue, $R_2$ is Na, K or $N(R_3)_3$, wherein $R_3$ is H, alkyl or hydroxyalkyl, $R_4$ is H, alkyl, hydroxyalkyl, $R_5$-COO-$R_2$, $[(CH_2)_nN]_x$-$R_5$-COO-$R_2$ or

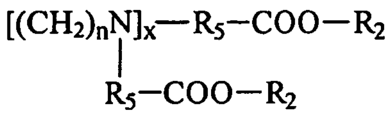

and $R_5$ is alkyl or hydroxyalkyl, n is an integer from 1 to 10 and x is an integer from 1 to 50;
b) a betaine of the general formula (II)

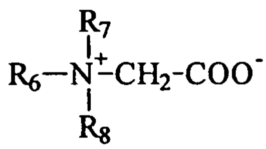

(II)

wherein $R_6$ is cocoalkyl, and $R_7$ and $R_8$ are hydroxyethyl;
c) a sulfobetaine corresponding to the general formula (III)

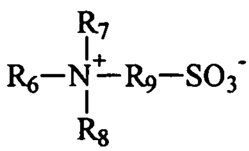

(III)

wherein $R_6$ is lauryl or cocoamidopropyl, $R_7$ and $R_8$ are methyl and $R_9$ is 2-hydroxypropyl;

**4.** The lubricant concentrate according to claim 3,
**characterized in that** the soap dispersant comprises an alkyl monoamine carboxylate corresponding to the general formula (I) wherein $R_1$ is cocoalkyl, $R_4$ is H, $R_5$ is ethylene and $R_2$ is triethanolammonium ion.

**5.** The lubricant concentrate according to claim 3,
**characterized in that** the soap dispersant comprises an alkyl polyamine carboxylate corresponding to the general formula (I), wherein $R_4$ is $[(CH_2)_nN]_x$-$R_5$-COO-$R_2$ and wherein $R_5$ is methylene or ethylene, preferably methylene; $R_2$ is Na or K, preferably Na; n is 2 to 4, preferably 3, and x is 1 to 5, preferably 1 to 3.

**6.** The lubricant concentrate according to any one of claims 3 to 5, **characterized in that** the amount of soap dispersant in the concentrate is larger than that of soap component.

**7.** The lubricant concentrate according to any one of claims 3 - 6, **characterized in that** the concentrate additionally contains an alkanolamine.

**8.** The lubricant concentrate according to any one of claims 3 to 7, **characterized in that** it contains at least one monovalent or multivalent alcohol as a dissolving promoter.

**9.** The lubricant concentrate according to any one of claims 3 to 8, **characterized in that** it contains a disinfactant or a preservative, such as 4,4-dimethyl oxazolidine, as a microbicidal component.

**10.** An aqueous lubricant composition, especially for belt or chain conveyors in the food industry, **characterized in that** it comprises the concentrate ingredients defined in any one of claims 3 to 9 plus a major amount of water.

**Patentansprüche**

**1.** Verwendung eines Schmiermittelkonzentrats zur Herstellung einer wässrigen Schmiermittelzusammensetzung für Band- oder Kettenförderer, die in der Lebensmittelindustrie verwendet werden, umfassend eine Seifenkomponente, insbesondere eine Alkali-Fettsäure oder Amino-Seife in einer Menge, die ausreichend ist, um der wässrigen Schmiermittelzusammensetzung schmierende Eigenschaften zu verleihen und gegebenenfalls umfassend geringe Mengen von weiteren handelsüblichen Schmiermittelkonzentrat-Komponenten, **dadurch** charakterisiert, dass das Konzentrat im wesentlichen frei von Komplexierungs- oder Sequestrationsmitteln für Härtebildner-Ionen ("hardness ion") ist und dass das Schmiermittelkonzentrat ein im wesentlichen bioabbaubares, nicht-ionisches, anionisches oder amphoteres Seifendispergiermittel umfasst, das die Ausfällung von schwer löslichen oder unlöslichen Verbindungen von Härtebildner-Ionen mit der Seifenkomponente verhindert, ohne wesentliche Komplexierungs- oder Sequestrationseigenschaften für Härtebildner-Ionen aufzuweisen, und dass das Schmiermittelkonzentrat in hartem Wasser verdünnt ist, wobei das Konzentrat als bioabbaubares Seifendispergiermittel mindestens ein Seifendispergiermittel ausgewählt aus den folgenden Verbindungen umfasst:

a) ein amphoteres Alkylmonoamin,
b) ein Polyamincarboxylat
c) ein Betain der allgemeinen Formel (II)

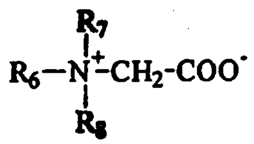

(II)

wobei $R_6$ Cocoalkyl ist und $R_7$ und $R_8$ Hydroxyethyl sind; oder
d) ein Sulfobetain entsprechend der allgemeinen Formel (III)

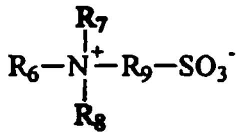

(III)

wobei $R_6$ Lauryl oder Cocoamidopropyl ist, $R_7$ und $R_8$ sind Methyl und $R_9$ ist 2-Hydroxypropyl;

**2.** Verwendung einer wässrigen Schmiermittelzusammensetzung gemäß Anspruch 1, wobei sie einen pH-Wert von 8 oder höher aufweist, um Band- oder Kettenförderer zu schmieren.

**3.** Schmiermittelkonzentrat, insbesondere zur Herstellung einer wässrigen Schmiermittelzusammensetzung für Band- oder Kettenförderer, die in der Lebensmittelindustrie verwendet werden, umfassend einer Seifenkomponente, insbesondere ein Alkali-Fettsäure oder Amino-Seife, in einer Menge, die ausreichend ist, um der wässrigen Schmiermittelzusammensetzung schmierende Eigenschaften zu verleihen und gegebenenfalls umfassend geringe Mengen von weiteren handelsüblichen Schmiermittelkonzentrat-Komponenten, charakterisiert **dadurch**, dass das Konzentrat im wesentlichen frei ist von Komplexierungs- oder Sequestrationsmitteln für Härtebildner-Ionen und ein im wesentlichen bioabbaubares, nicht-ionisches, anionisches oder amphoteres Seifendispergiermittel umfasst, das die Ausfällung von schwerlöslichen oder unlöslichen Verbindungen von Härtebildner-Ionen der Seifenkomponente verhindert, ohne wesentliche Komplexierungs- oder Sequestrationseigenschaften für Härtebildner-Ionen aufzuweisen, wobei das Seifendispergiermittel ausgewählt wird aus den folgenden Verbindungen:

a) mindestens eine Komponente der allgemeinen Formel (I)

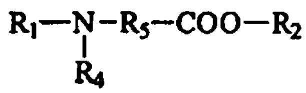

(I)

wobei $R_1$ ein unverzweigter, verzweigter, gesättigter oder ungesättigter Alkylrest ist, der von 8 bis 22 Kohlenstoffatomen aufweist oder ein entsprechender Alkoxyrest, $R_2$ ist Na, K oder $N(R_3)_3$, wobei $R_3$ H, Alkyl oder Hydroxyalkyl ist, $R_4$ ist H, Alkyl, Hydroxyalkyl, $R_5$-COO-$R_2$, $[(CH_2)_nN]_x$-$R_5$-COO-$R_2$ oder

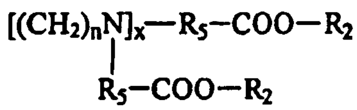

und $R_5$ ist Alkyl oder Hydroxyalkyl, n ist eine Zahl von 1 bis 10 und x ist eine Zahl von 1 bis 50;
b) ein Betain der allgemeinen Formel (II)

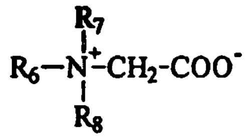

(II)

wobei $R_6$ Cocoalkyl ist und $R_7$ und $R_8$ Hydroxyethyl sind;
c) ein Sulfobetain entsprechend der allgemeinen Formel (III)

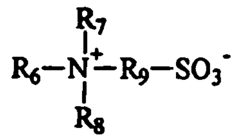

(III)

wobei $R_6$ Lauryl oder Cocoamidopropyl ist, $R_7$ und $R_8$ sind Methyl und $R_9$ ist 2-Hydroxypropyl;

**4.** Schmiermittelkonzentrat gemäß Anspruch 3,
**dadurch** charakterisiert, dass das Seifendispergiermittel ein Alkylmonoamincarboxylat entsprechend der allgemeinen Formel (I) umfasst, wobei $R_1$ Cocoalkyl ist, $R_4$ H ist, $R_5$ Ethylen und $R_2$ ein Triethanolammonium-Ion ist.

**5.** Schmiermittelkonzentrat gemäß Anspruch 3,
**dadurch** charakterisiert, dass das Seifendispergiermittel ein Alkylpolyamincarboxylat umfasst, entsprechend der allgemeinen Formel (I), wobei $R_4$ $[(CH_2)_nN]_x$-$R_5$-COO-$R_2$ ist und wobei $R_5$ Methylen oder Ethylen ist, vorzugsweise Methylen; $R_2$ Na oder K ist, vorzugsweise Na; n 2 bis 4 ist, bevorzugt 3 und x 1 bis 5 ist, bevorzugt 1 bis 3.

**6.** Schmiermittelkonzentrat gemäß einem der Ansprüche 3 bis 5,
**dadurch** charakterisiert, dass die Menge an Seifendispergiermittel in dem Konzentrat größer ist als die der Seifenkomponente.

**7.** Schmiermittelzusammensetzung gemäß einem der Ansprüche 3 bis 6, **dadurch** charakterisiert, dass das Konzentrat zusätzlich ein Alkanolamin enthält.

**8.** Schmiermittelkonzentrat gemäß einem der Ansprüche 3 bis 7,
**dadurch** charakterisiert, dass es mindestens einen mono-valenten oder multi-valenten Alkohol als Lösungspromoter ("dissolving promoter") enthält.

**9.** Schmiermittelkonzentrat gemäß einem der Ansprüche 3 bis 8,
**dadurch** charakterisiert, dass es ein Desinfektionsmittel oder ein Konservierungsmittel, wie z.B. 4,4-Dimethyloxazolidin als eine mikrobizide Komponente enthält.

**10.** Schmiermittelzusammensetzung, insbesondere für Band- und Kettenförderer in der Lebensmittelindustrie,
**dadurch** charakterisiert, dass es die Konzentratbestandteile, die in irgendeinem der Ansprüche 3 bis 9 definiert sind und zusätzlich eine große Menge Wasser umfasst.

## Revendications

**1.** Utilisation d'un concentré lubrifiant pour préparer une composition lubrifiante aqueuse pour des convoyeurs à courroie ou chaîne utilisés dans l'industrie alimentaire, comportant un composant de savon, en particulier un savon

alcalin ou amino d'acide gras, en une quantité suffisante pour fournir des propriétés lubrifiantes à la composition lubrifiante aqueuse, et, dans ce cas, comportant des quantités mineures d'autres composants de concentré lubrifiant classiques,
**caractérisée en ce que** le concentré est essentiellement exempt d'agents complexants ou séquestrants d'ions de dureté, et **en ce que** le concentré lubrifiant comporte un dispersant de savon non-ionique, anionique ou amphotère essentiellement biodégradable empêchant la précipitation de composés ioniques de dureté faiblement solubles ou insolubles du composant de savon sans avoir de quelconques propriétés complexantes ou séquestrantes d'ions de dureté, et **en ce que** le concentré lubrifiant est dilué dans de l'eau dure, le concentré comportant, en tant que dispersant de savon biodégradable, au moins un dispersant de savon sélectionné parmi les composés suivants :

a) une alkylmonoamine amphotère,
b) un carboxylate de polyamine,
c) une bétaïne ayant la formule générale (II)

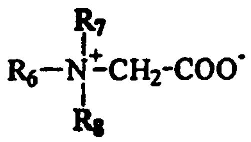

**(II)**

où $R_6$ est un cocoalkyle, et $R_7$ et $R_8$ sont un hydroxyéthyle, ou
d) une sulfobétaïne correspondant à la formule générale (III)

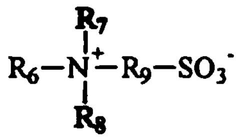

**(III)**

où $R_6$ est un lauryle ou cocoamidopropyle, $R_7$ et $R_8$ sont un méthyle et $R_9$ est un 2-hydroxypropyle.

**2.** Utilisation d'une composition lubrifiante aqueuse selon la revendication 1, et ayant un pH égal à 8 ou plus, pour lubrifier une courroie ou une chaîne de convoyeur.

**3.** Concentré lubrifiant, en particulier pour préparer une composition lubrifiante aqueuse pour des convoyeurs à courroie ou chaîne utilisés dans l'industrie alimentaire, comportant un composant de savon, en particulier un savon alcalin ou amino d'acide gras, en une quantité suffisante pour fournir des propriétés lubrifiantes à la composition lubrifiante aqueuse et, dans ce cas, comportant des quantités mineures d'autres composants de concentré lubrifiant classiques, **caractérisé en ce que** le concentré est essentiellement dépourvu d'agents complexants ou séquestrants d'ions de dureté, et comporte un dispersant de savon non-ionique, anionique ou amphotère essentiellement biodégradable empêchant la précipitation de composés ioniques de dureté faiblement solubles ou insolubles du composant de savon sans avoir de quelconques propriétés complexantes ou séquestrantes d'ions de dureté, dans lequel le dispersant de savon est sélectionné parmi les composés suivants :

a) au moins un composé ayant la formule générale (I)

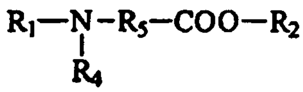

**(I)**

où $R_1$ est un résidu alkyle linéaire, ramifié, saturé ou insaturé ayant 8 à 22 atomes de carbone ou un résidu alkoxy correspondant, $R_2$ est Na, K ou N $(R_3)_3$, où $R_3$ est H, un alkyle ou un hydroxyalkyle, $R_4$ est H, un alkyle, un hydroxyalkyle, $R_5$-COO-$R_2$, $[(CH_2)_nN]_x$-$R_5$-COO-$R_2$ ou

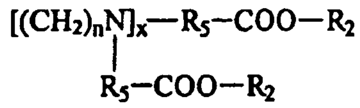

et $R_5$ est un alkyle ou un hydroxyalkyle, n est un nombre entier compris entre 1 et 10 et x est un nombre entier compris entre 1 et 50,
b) une bétaïne ayant la formule générale (II)

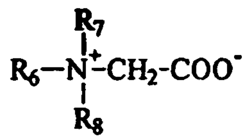

**(II)**

où $R_6$ est un cocoalkyle, et $R_7$ et $R_8$ sont un hydroxyéthyle,
c) une sulfobétaïne correspondant à la formule générale (III)

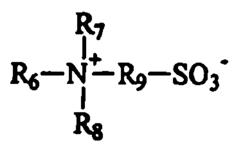

**(III)**

où $R_6$ est un lauryle ou cocoamidopropyle, $R_7$ et $R_8$ sont un méthyle et $R_9$ est un 2-hydroxypropyle.

**4.** Concentré lubrifiant selon la revendication 3,
**caractérisé en ce que** le dispersant de savon comporte un carboxylate d'alkylmonoamine correspondant à la formule générale (I) où $R_1$ est un cocoalkyle, $R_4$ est H, $R_5$ est un éthylène et $R_2$ est un ion triéthanolamonium.

**5.** Concentré lubrifiant selon la revendication 3,
**caractérisé en ce que** le dispersant de savon comporte un carboxylate d'alkylpolyamine correspondant à la formule générale (I), où $R_4$ est $[(CH_2)_nN]_x$-$R_5$-COO-$R_2$ et où $R_5$ est un méthylène ou éthylène, de préférence un méthylène, $R_2$ est Na ou K, de préférence Na, n est compris entre 2 et 4, de préférence égal à 3, et x est compris entre 1 et 5, de préférence entre 1 et 3.

**6.** Concentré lubrifiant selon l'une quelconque des revendications 3 à 5, **caractérisé en ce que** la quantité de dispersant de savon dans le concentré est supérieure à celle du composant de savon.

**7.** Concentré lubrifiant selon l'une quelconque des revendications 3 à 6, **caractérisé en ce que** le concentré contient de plus une alkanolamine.

**8.** Concentré lubrifiant selon l'une quelconque des revendications 3 à 7, **caractérisé en ce qu'**il contient au moins un alcool monovalent ou polyvalent en tant que promoteur de dissolution.

**9.** Concentré lubrifiant selon l'une quelconque des revendications 3 à 8, **caractérisé en ce qu'**il contient un désinfectant ou un conservateur, tel que 4,4-diméthyloxazolidine, en tant que composant microbicide.

**10.** Composition lubrifiante aqueuse, en particulier pour des convoyeurs à courroie ou chaîne dans l'industrie alimentaire, **caractérisée en ce qu'**elle comporte les ingrédients de concentré définis selon l'une quelconque des revendications 3 à 9 plus une quantité majeure d'eau.

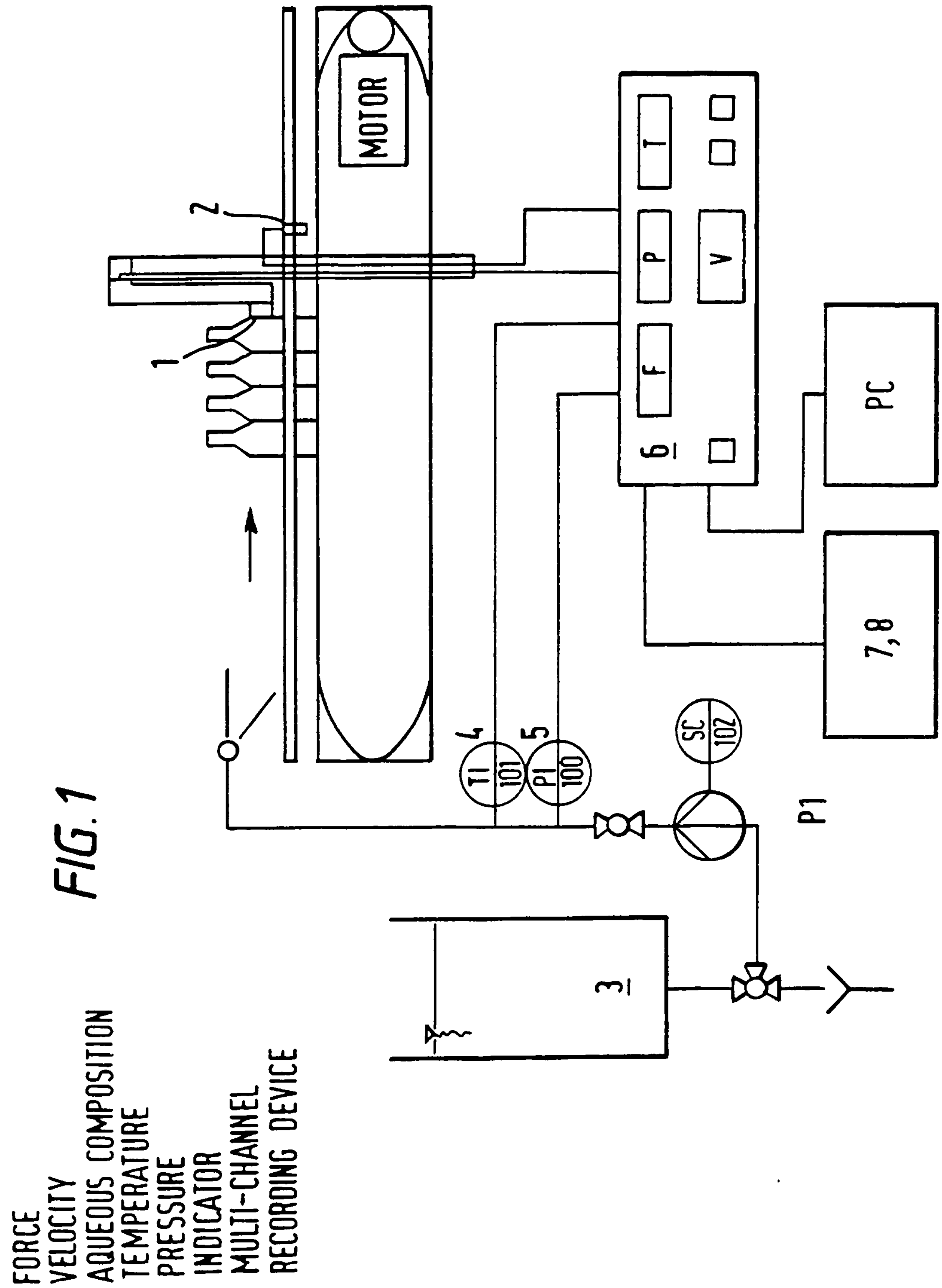
1 FORCE
2 VELOCITY
3 AQUEOUS COMPOSITION
4 TEMPERATURE
5 PRESSURE
6 INDICATOR
7 MULTI-CHANNEL
8 RECORDING DEVICE
MOTOR
F
P
T
V
6
PC
7,8
TI 101
PI 100
SC 102
P1
3

FIG. 1

FIG. 2

SLIDING FRICTION COEFFICIENT OF 0.2% APPLICATION SOLUTIONS